# EUROPEAN PATENT SPECIFICATION

(11) **EP 3 701 537 B1**
(45) Date of publication and mention of the grant of the patent: **30.08.2023**
(21) Application number: 18796862.3
(22) Date of filing: 23.10.2018
(51) Int. Cl.: G16H 20/70, A61B 5/16

(54) **COMPUTING SYSTEM AND METHOD FOR TREATING OF MOOD-RELATED DISORDERS**
RECHNERSYSTEM UND VERFAHREN ZUR BEHANDLUNG VON AFFEKTIVEN STÖRUNGEN
SYSTÈME INFORMATIQUE ET PROCÉDÉ DE TRAITEMENT DES TROUBLES LIÉS À L'HUMEUR

(30) Priority: 23.10.2017 LU 100492
(43) Date of publication of application: 02.09.2020
(73) Proprietor: Neuraltrain GmbH, 10117 Berlin (DE)
(72) Inventor: LAUTNER, Christian, 10117 Berlin (DE); CHAKRABARTY, Arnab, 13357 Berlin (DE); MÜSCHENICH, Markus, 10117 Berlin (DE)
(74) Representative: Harrison, Robert John
(86) International application number: PCT/EP2018/079000
(87) International publication number: WO 2019/081487

(56) References cited:
- US-A1- 2003 109 800
- US-A1- 2014 017 645
- M. BAUER ET AL: "Attentional Modulation of Alpha/Beta and Gamma Oscillations Reflect Functionally Distinct Processes", THE JOURNAL OF NEUROSCIENCE, vol. 34, no. 48, 26 November 2014 (2014-11-26), pages 16117-16125, XP055489608, US ISSN: 0270-6474, DOI: 10.1523/JNEUROSCI.3474-13.2014

## Description

### Cross-Relation to Other Applications

The invention claims priority of Luxembourg Patent Application No. 100492 filed on 23 October 2017.

### Field of the Invention

The invention relates to a computing system and a method for treating mood-related disorders.

### Background of the Invention

Depression is fast becoming the leading cause of illness and disability. The World Health Organisation (WHO), in its factsheet on depression, estimates that more than 300 million people are affected by depression. A more recent report puts the number at 350 million. About a quarter of the affected population is believed to be in Europe. With industrialisation and globalisation, we are seeing an increase in incidents of depression and use of anti-depressives.

According to the WHO, in the European region, 1 out of 15 citizens suffer or have suffered from major depression and this becomes 4 out of 15 citizens when all forms of depression and anxiety disorders are included. Less than half of those affected by depression have access to effective treatment (and in some countries this figure is as low as 10%). Lastly, the WHO mentions that 3 out of 4 patients diagnosed with depression in the European region do not receive adequate treatment.

Increasing the ease of access and empowerment of patients in healthcare can play a huge role in correcting this lack of adequate treatment. Currently, geographical isolation, lack of resources and lack of a holistic solution are all factors leading to the aforementioned lack of access to treatment. This lack of access can be addressed to a significant degree using digital health solutions addressing mental health issues, such as mood-related disorders, including clinical depression and other mood-related disorders.

One demographic suffering from this illness is the adolescent population. Adolescent depression is growing in numbers with contributing factors like peer pressure, societal anxiety, increasing isolation in an increasingly digital world, etc. This demographic is particularly relevant for therapy, as an adolescent brain is far more malleable to re-modelling than an adult brain.

Another demographic suffering from depression is the elderly population above the age of 60. Evidence suggests that mood-related disorders including depression are on the rise in such demographics given the rise of nuclear families, increasing urban living resulting in increased isolation and an increasingly automated digital society.

Lastly, there is another demographic to be considered: many people suffer from mood-related disorders, and do not meet the strict clinical criteria for depression. These people exhibit some depressive symptoms, but do not meet the criteria for a depressive disorder. This condition is named "sub-clinical depression". Such sub-threshold depression considerably affects the quality of life and may predispose a person to later development of a major depressive disorder.

The economic costs of all these patients in the healthcare system and employment system means increased spending on healthcare per patient and lost working days costing businesses their productivity. Currently, 50% of chronic sick leave in the EU is due to depression. In fact, the current economic costs of mood and anxiety related disorders along with depression account for around costs of 170 billion Euros annually. The human cost of suffering (disease burden) is illustrated by another WHO report (Health for the World's Adolescents), which reports depression (Unipolar Depressive Disorder) to be the leading cause of disability-adjusted life years (DALYs) lost globally.

Given the above background, it will be understood that there are various factors contributing to an unmet medical need in the field for the treatment of depression. There is a need to provide remote administration of an effective, lasting and sustainable solution that can also cater to the demographics listed above and can also provide a therapy that can span across all three pillars of healthcare: prevention, therapy/management and remission. To sum up, the contributing factors in the unmet need mentioned here are:
- Lack of resources available to cater to all patients- both doctors and therapists
- Long waiting times in having recourse to specialists
- Lack of therapy solutions for patients of sub-clinical depression, mood and anxiety disorders, milder forms of depression as they are not always the ideal candidates for pharmacotherapy
- Lack of immediate access to specialists in sub-urban and rural areas of Europe
- Long intervals between therapy sessions
- Expense factor, if patients wants more therapy sessions or smaller inter-therapy intervals.
- No therapy-customisation to individual patients and patient demographic stratification
- Therapy success is greatly dependent on patient-therapist personal chemistry

A number of patent applications are known which describe the use of computing systems for diagnosis. For example, International Patent Application No. WO 2016/69058 (General Hospital Corp / MIT) teaches a system and method for diagnosing mental or emotional disorders. A series of input data related to activity in the brain of the patient is acquired whilst the patient performs a battery of behavioural task. The system identified what is abnormal for that individual patient from examination of the data. WO '058 does not teach a therapeutic method.

US Patent Application 2003/109800 teaches a computer system for improving visual perception disorders, such as amblyopia, myopia, hyperopia, presbyopia, super-normal vision, or dyslexia. The system achieves this by generating a succession of so-called visual perception task images which the user must select. Subsequently generated ones of the images are based at least in part of the user's perception of one or more previously presented images. The generation and modification of the images is repeated until at least one characteristic of the person's visual perception capabilities is evaluated or improved. The described embodiments relate principally to the treatment of amblyopia.

US patent application publication number US 2014/017645 relates to a trainer for enhancing the attention of a user the system described and requires the user to interact with the system. The trainer includes a processor couple to a display device on which a cognitive task is presented to the user. The processor assesses the attentional control of the user in relation to the cognitive task and generates instructions and/or a feedback based on the attentional control assessment.

### Summary of The Invention

The aim and scope of this invention is to implement an innovative and disruptive medical solution that aims to provide medical treatment of different forms of mood-related disorders and make the solution user or patient-centric. The proposed solution can be independent of a therapist and doctor and substantially independent of geographic location/isolation. Finally, the proposed solution aims to administer a "software as a drug" solution as a part of a digital therapeutic continuum in treatment of depression and other mood-related disorders, including affective-spectrum disorders. In the context of this disclosure, the term "mood-related disorder" is intended to encompass not only sub-clinical depression, but also depression itself and anxiety-related disorders.

This document discloses a solution comprising a computing system, a method and a computer program product for treatment of mood-related disorders. The computing system comprises one or more processors, memory, a display device and one or more programs. The one or more programs are stored in the memory and are configured to be executed by the one or more processors to treat a subject suffering from a mood-related disorder. The programs are configured to display a first image on the display device for a first period of time (for example for 500 milliseconds to 2 seconds), wherein the first image is adapted to induce gamma oscillations in the subject's brain; and display, for a second period of time, a second image on the display device after the first period of time, wherein the second image comprises positive-valence laden content.

In this context, the term "gamma oscillations" is used to mean a pattern of neural oscillation in a human being with a frequency of between 25 Hz and 140 Hz (Colgin, 2011a) and which is typically, but not limited to, between 35 Hz and 70 Hz.

In one aspect of the invention, the display of the first image and the display of the second image are repeated over several cycles.

The system enables the treatment of mood-related disorders, such as depression, in all its clinical and sub-clinical forms.

The first image is, for example, a Gabor patch. The second image is chosen from a set of images enjoyed by the viewer.

A method for treatment of mood-related disorders, including depression, is also disclosed. The method comprises displaying a first image on the display device for a first period of time, wherein the first image is adapted to induce gamma oscillations in the viewer's brain; and displaying, for a second period of time, a second image on the display device after the first period of time, wherein the second image comprises content appropriate to the mood-related disorder.

This method and apparatus described in this document fit in the evolving age of a digital society. As the society becomes increasingly digital, the aim should be to provide therapies using tools that the patients are likely to already have as a part of their lifestyle, i.e. digital, and whose efficacy or patient outcome can be measured in a way which disrupts the patients' lifestyles the least. It also is a step in the direction of establishing a digital therapeutic continuum in the therapeutic care and management of depression and other mood-related disorders.

The method and system described in this document is intended to be carried out independently of intervention by a medical doctor or therapist. The method and system, however, can be integrated in the existing healthcare system and in the established therapy models of depression or other mood-related disorders very well. This is because once the medical doctor has seen the patient and diagnosed his or her condition, the medical doctor can recommend the solution to those patients who display depressive symptoms but are not clinically depressed or to those with mood-related disorders both as a part of therapy as well as remission/prevention.

The system and method provide alternatives to conventional treatment modules of mood-related disorders include pharmacotherapy (which is not a long-term solution given that patients develop resistance to the drugs and chronic dependence on these drugs can lead to severe withdrawal consequences) and/or psychotherapy in the form of Cognitive Behavioural Therapy, CBT (a more holistic, sustainable therapy). The proposed solution fits very well in the established parameters of CBT as the method and system use some elements of CBT. However, since the solution requires only the patient to self-administer, the dependence on the availability of the medical doctor or the therapist is eliminated, thus potentially reducing suffering of the patient and economic burden of the disease. Additionally, the independence from restrictions of geographical location further supports the benefits mentioned above.

The solution can be further used to optimise patient response to face-to-face or internet-based CBT sessions, in between therapy sessions and/or whenever necessary by the patients. It can also be used every day and anywhere independent of the situation in which the patient finds himself or herself.

The proposed solution is customisable. The solution is not a "one-size fits all". Instead, the patient's preferences define the therapy and makes each patient' software-pill unique to that patient.

### Description of the Figures

Fig. 1 shows an example of the system of the invention.
Fig. 2 shows an outline of the method of the invention.
Fig. 3 shows an example of a Gabor patch.

### Detailed Description of the Invention

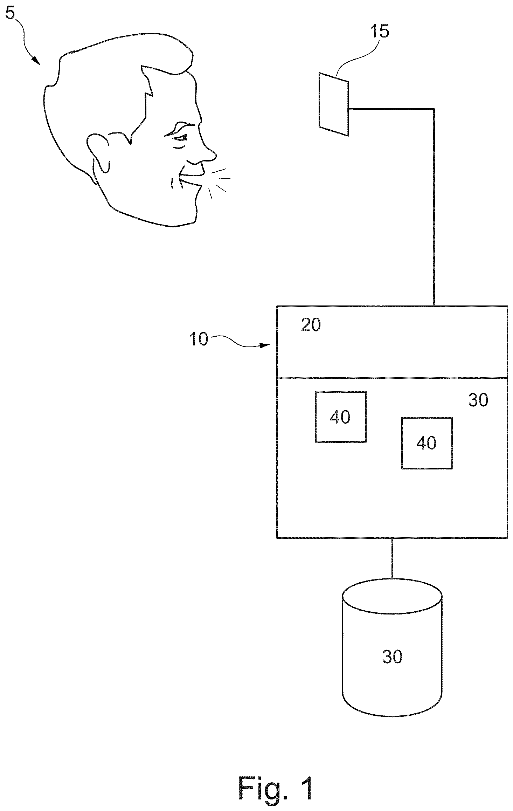

Fig. 1 shows an outline of a computing system 10 used for the implementation of the method disclosed in this document. It will be appreciated that this is merely a simplified overview and is not intended to be limiting of the invention. The computing system 10 comprises one or more processors 20 and memory 30. A display device 15 is connected to the one or more processors 20 either by a cable or wirelessly. The display device 15 can be a monitor, smartphone, tablet or similar device and is viewed by a viewer 5. The display device 15 can be a hand-mounted device. The viewer 5 would be typically a patient, a subject, a medical practitioner or any other user of the computer system 10.

The one or more programs 40 are stored in the memory 30 and are configured to be executed by the one or more processors 20 to treat a subject (the viewer 5) suffering from a brain dysfunction, such as a mood-related disorder. In one aspect of the disclosure, the one or more programs 40 are configured to display a first image on the display device 15 for a first period. This first image is adapted to induce gamma oscillations in the brain of the viewer 5. Subsequently, for a second period of time, a second image on the display device 15 after the first period of time. The second image comprises content which is appropriate to the brain dysfunction being treated. In one aspect, the content is positive valence-laden content used for treating the mood-related disorder.

The computing system 10 can be used, for example, to treat a mood-related disorder, as discussed above. Symptoms of clinical depression and other mood-related disorders are listed in detail in medical literature. In several forms of depression, patients display a characteristically common pattern: decreased use of mental imagery with an increased use of observer perspective, increased rumination (worrying or brooding) and negative self-evaluation as well as an overall decreased positive affect. It is known, for example, that depressed people show less positive mood or emotion compared to healthy individuals (Grol et al., 2017). The provision of the first image is designed to induce gamma oscillations that will drive attention and subsequently neuroplasticity associated with the following positive valence content, leading to reinforced positive mental imagery.

In this context, the term "observer perspective" is used to indicate the opposite of a field perspective. If asked to imagine a situation using mental imagery and then explain the situation, an adopter of the observer perspective will describe the situation as a passive observer. In contrast, the adopter of the field perspective will describe the situation as someone who is present in the scene playing an active role in the situation. Rumination is the focused attention on the symptoms of the patient's distress, and on the possible causes and consequences, of the illness as opposed to its solutions. Both rumination and worry are associated with anxiety and other negative emotional states, which can lead to further depression. Neuroplasticity is the ability of the cells of the brain (neurons) to change or be altered throughout the life of an individual.

So-called cognitive behavioural therapy (CBT) has proven to be a useful tool to treat depression in the long term. There is considerable evidence for the efficacy of CBT in many forms of depression, anxiety, and other mood-related disorders and many from the affective disorders spectrum. One aspect of the CBT is the use of positive mental imagery or positive mood induction (Holmes et al., 2007; Holmes and Mathews, 2010). However, given that depressed people have a reduced positive affect and an increased negative imagery related emotion (Clasen et al., 2013)(Holmes et al., 2008), the efficacy of this treatment procedure is limited, as the positive imagery fails to generate a change in the neural patterns in depressed brains that arrange for such imagery associations. The proposed method and computing system attempt to solve this problem by deriving its concept and its strength from a combinatorial approach: using neuroscientific methods of brain plasticity to remodel and reinforce synaptic weights and neural connectomes to increase and strengthen positive valence neural circuitry in patients of mood-related disorders.

It is known that networks of brain cells synchronise their activity at certain frequencies depending on the state of arousal and the task at hand. Such synchronised activity is called neuronal oscillation and happens at alpha, beta, gamma, delta and theta ranges. Each of these ranges are distinct frequency ranges associated with different functions or tasks. Oscillations in the gamma range, for example, refer to periodic fluctuations in the local field potential of a neuronal structure, or of an ensemble of neurons forming an assembly, at rates ranging from 35-70 Hz. The actual frequency ranges can be as low as 30 Hz to as high as 140 Hz as reported in different studies but it is the "slow" or "narrowband" gamma of 35-70 Hz (Colgin, 2011b, 2015; Sedley and Cunningham, 2013) that has received widespread attention and has been implicated in cognition, perception, selective attention, binding of relevant features of a stimulus set (the binding problem), etc (Perry et al., 2013; Van Pelt and Fries, 2013). There is strong evidence that synchronous gamma activity provides a perceptual augmentation of relevant stimulus features over non-relevant stimuli features (Nyhus and Curran, 2010). Such intrinsic brain rhythm is not only spontaneous or activity-dependent but can also be entrained from the outside by various means of neural stimulation.

Several studies have documented evidence on the multitude of ways of induction of oscillatory activity in various regions of the brain contingent on the stimulus used (Walker et al., 2009; Bauer et al., 2014). In psychophysical studies on attention, and in particular, selection and visual attention, stimuli comprising of high contrast visual gratings, such as Gabor patches or similar, have been used to generate gamma frequency oscillations in human and animal visual cortex (Walker et al., 2009; Bauer et al., 2014).

The developers of the computer system and method have recognised that external entrainment of gamma frequency oscillations can be exploited to direct attention to positive valence imagery when such positive valence imagery is presented during the temporal window of visual grating-induced gamma band activity. It is envisaged that repeated gamma band activity in an assembly of neurons leads to synaptic plasticity and hence, to synaptic weight changes in those neurons. By combining and directing such synaptic weight changes to be associated with positive imagery, the method of this disclosure exploits the ability of the brain to retrain and remodel its brain circuits. The latter concept is also termed "plasticity".

The plasticity of neurons, networks of neurons or the brain on the whole is supported by a mountain of evidence since the discovery of the cellular basis of plasticity in the 1970s by Bliss, Lomo and Andersen (Bliss and Lomo, 1970, 1973, Lomo, 2003, 2017). Their work provided the formal experimental proof for this phenomenon, which was theoretically formulated by the legendary psychologist Donald Hebb in 1949, called Hebb's postulate (Hebb, 2002).

Positive mood inductions and mental imagery re-scripting is an accepted part of CBT in mood-related and anxiety disorders as well as depression. Several academic, scientific studies have gathered evidence on the efficacy of cognitive behavioural therapy and have attempted to illustrate the differences between healthy and depressed or dysphoric subjects in perceiving and reacting to mental imagery. To appreciate the mechanism of action of the proposed therapy method, it is necessary to look at the underlying recurring behavioural patterns of depression and dysphoria patients:
- Decreased use of mental imagery
- Increased use of an observer-perspective
- Increased rumination
- Increased negative self-evaluation
- Decreased overall positive-affect

Providing the last missing link to all these aspects of the proposed new therapy is that the neural circuitry that is thought to underlie such behavioural patterns in these patients is altered when compared to healthy subjects. Scientific studies have looked at and have illustrated these circuit differences between the brains of depressed and healthy individuals (Osuji and Cullum, 2005; Sachs et al., 2007; Cheng et al., 2016; Makovac et al., 2016; Canali et al., 2017). Evidence exists that there are reduced number of connections in the positive-emotion and reward-related circuits of the brain of depression patients, while negative-emotion and punishment-related circuits of the brain present increased connections in the brains of depression patients while compared to healthy individuals (Cheng et al., 2016). Adding to the evidence are studies that have shown that the amplitude and duration of gamma band oscillations in depressed individuals are reduced in depression patients (Canali et al., 2017).

The method and the computing system propose to attempt a change in the underlying brain circuits and their network properties that will cause a re-training and strengthening of mental imagery and positive-affect perception of depression patients. It is envisaged that, with regular use, such brain circuits can be trained and strengthened as one changes neuronal and neuro-muscular connections with regular mental and physical training.

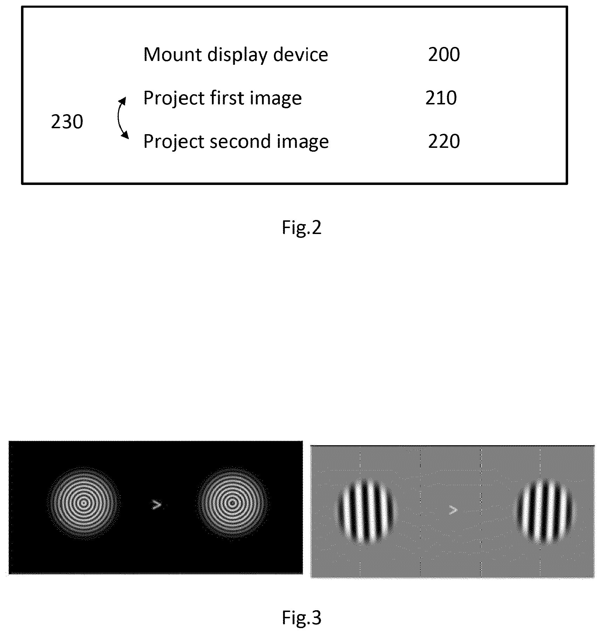

Fig. 2 shows an outline of the method. In a first step 200, a display device 15 can be mounted on a support for viewing by the viewer 5. A first image is produced on the display device 15 by one of the computer programs 40 for a first period of time and the viewer 5 looks at this displayed first image. The method uses the Gabor-patch technique, and variants thereof, to induce gamma band oscillations in the visual-cortical areas of the brains of patients suffering from a mood-related disorder temporarily. The method is non-invasive and is achieved using optimised visual patterns shown to the eyes of the viewer 5 and thence to the brain using the display device 15.

In a narrow window of time, gamma band activity is thus induced in the brain and in step 220, a second image is projected on the display device 15. This second image has a positive valence, and could be a nature photo, an animal photo, or any image known to produced positive thoughts in the brain of the viewer 5.

Steps 210 and 200 are repeated several times, as is indicated by arrow 230, during a single session. Such single sessions can then be repeated several times daily and on a continual basis. The viewer 5 will have the option to select the content as per his or her preferences or positive valence. The latter is important because this utilises the innate memories of the patient and his or her associations of the content with good times in his or her life. Furthermore, this reduces the risk of therapy resistance, as some content that is of positive valence to one viewer might be of neutral or even negative-valence to others. This way, the therapy is tailored to individual needs of the patient and is a step towards precision medicine. Finally, the patient, in this method, becomes an important stakeholder in the process of treatment and hence, can potentially make the therapy much more effective in terms of patient outcome.

The proposed therapy has particularly high potential in the following patient demographics:
- Adolescents, who are the best fit for any kind of synaptic and/or network plasticity.
- Sub-clinical depression and mood-related disorder sufferers along with mild depression patients could be highly responsive due to less cemented circuitry reinforcing depressed behavioural pattern
- Senior patients could be highly responsive because this therapy provides them with a highly immersive therapy experience that addresses their boredom in an increasingly isolated social environment as well as higher attention-span to the content base. Also, content with positive valence would be rich and diverse for them.
- In people with social difficulties or lacking motivation to venture out during depression, this therapy offers anonymity and no requirement to venture out or be in presence of other people. Therapy non-adherence or termination among others are also caused by self-isolation and lack of drive and discomfort in front of people, defining features in depression. Our mobile, digital therapy eliminates the barriers mentioned above.

## Claims

1. A computing system (10) for treatment of a mood-related disorder comprising:
one or more processors (20);
memory (30);
a display device (15); and
one or more programs (40), wherein the one or more programs (40) are stored in the memory (30) and are configured to be executed by the one or more processors (20) to treat a viewer (5) suffering from the mood-related disorder, the one or more programs (40) being configured to
i) display a first image on the display device (15) for a first period of time, wherein the first image is adapted to induce gamma oscillations in a brain of the viewer (5); and
ii) displaying after the inducing of the gamma oscillations, for a second period of time, a second image on the display device (15) after the first period of time, wherein the second image comprises positive valence-laden content appropriate to the treatment of the mood-related disorder.

2. The computing system of claim 1, wherein the display of the first image and the display of the second image are repeated over several cycles.

3. The computer system of any of the above claims, further comprising measuring devices to measure activity of the brain.

4. The computer system of any of the above claims, wherein the first image is a Gabor patch or a variant thereof.

5. The computer system of any of the above claims, wherein the second image is one of a
nature photo or an animal photo.

6. A computer program product for treatment of a mood-related disorder comprising:
a first program logic for causing a display of a first image on a display device (15) for a first period of time, wherein the first image is adapted to induce gamma oscillations in a brain of a viewer (5); and
a second program logic for causing a display after the inducing of the gamma oscillations, for a second period of time, a second image on the display device (15) after the first period of time, wherein the second image comprises positive valence-laden content appropriate to the treatment of the mood-related disorder.

## Patentansprüche

1. Computersystem (10) zur Behandlung einer stimmungsbezogenen Störung umfassend:
einen oder mehrere Prozessoren (20);
einen Speicher (30);
eine Anzeigevorrichtung (15); und
ein oder mehrere Programme (40), wobei das eine oder die mehreren Programme (40) in dem Speicher (30) gespeichert ist/sind und eingerichtet ist/sind, um von dem einen oder den mehreren Prozessoren (20) ausgeführt zu werden, um einen Betrachter (5) zu behandeln, welcher an der stimmungsbezogenen Störung leidet, wobei das eine oder
die mehreren Programme (40) eingerichtet ist/sind, um
i) ein erstes Bild auf der Anzeigevorrichtung (15) für eine erste Zeitspanne anzuzeigen, wobei das erste Bild geeignet ist, um Gamma-Oszillationen in einem Gehirn des Betrachters (5) zu induzieren; und
ii) ein zweites Bild auf der Anzeigevorrichtung (15) nach dem Induzieren der Gamma-Oszillationen für eine zweite Zeitspanne anzuzeigen, wobei das zweite Bild einen positiven valenzbeladenen Inhalt umfasst, der für die Behandlung der stimmungsbezogenen Störung geeignet ist.

2. Computersystem nach Anspruch 1, bei welchem die Anzeige des ersten Bildes und die Anzeige des zweiten Bildes über mehrere Zyklen wiederholt wird.

3. Computersystem nach einem der vorstehenden Ansprüche, ferner umfassend Messgeräte zur Messung einer Aktivität des Gehirns.

4. Computersystem nach einem der vorstehenden Ansprüche, bei welchem das erste Bild ein Gabor-Patch oder eine Variante hiervon ist.

5. Computersystem nach einem der vorstehenden Ansprüche, bei welchem das erste Bild eines von einem Naturfoto oder einem Tierfoto ist.

6. Computerprogrammprodukt zur Behandlung einer stimmungsbezogenen Störung umfassend:
eine erste Programmlogik, um eine Anzeige eines ersten Bildes auf einer Anzeigevorrichtung (15) für eine erste Zeitspanne zu veranlassen, wobei das erste Bild geeignet ist, um Gamma-Oszillationen in einem Gehirn eines Betrachters (5) zu induzieren; und eine zweite Programmlogik, um eine Anzeige eines zweiten Bildes auf der Anzeigevorrichtung (15) nach dem Induzieren der Gamma-Oszillationen für eine zweite Zeitspanne zu veranlassen, wobei das zweite Bild einen positiven valenzbeladenen Inhalt umfasst, der für die Behandlung der stimmungsbezogenen Störung geeignet ist.

## Revendications

1. Système informatique (10) pour le traitement d'un trouble lié à l'humeur comprenant :
un ou plusieurs processeurs (20);
une mémoire (30);
un dispositif d'affichage (15); et
un ou plusieurs programmes (40), dans lequel le ou les programmes (40) sont stockés dans la mémoire (30) et sont configurés pour être exécutés par le ou les processeurs (20) pour traiter un spectateur (5) souffrant du trouble lié à l'humeur, le ou les programmes (40) étant configurés pour
i) afficher une première image sur le dispositif d'affichage (15) pendant une première période de temps, la première image étant adaptée pour induire des oscillations gamma dans le cerveau du spectateur (5) ; et
ii) afficher, après l'induction des oscillations gamma, pendant une deuxième période de temps, une deuxième image sur le dispositif d'affichage (15) après la première période de temps, la deuxième image comprenant un contenu à valence positive approprié au traitement du trouble lié à l'humeur.

2. Système informatique selon la revendication 1, dans lequel l'affichage de la première image et l'affichage de la deuxième image sont répétés sur plusieurs cycles.

3. Système informatique selon l'une quelconque des revendications précédentes, comprenant en outre des appareils de mesure pour mesurer l'activité du cerveau.

4. Système informatique selon l'une quelconque des revendications précédentes, dans lequel la première image est un patch de Gabor ou une variante de celui-ci.

5. Système informatique selon l'une quelconque des revendications précédentes, dans lequel la deuxième image est l'une parmi une photo de nature ou une photo d'animal.

6. Produit de programme d'ordinateur pour traitement d'un trouble lié à l'humeur comprenant :
une première logique de programme pour provoquer un affichage d'une première image sur un dispositif d'affichage (15) pendant une première période de temps, la première image étant adaptée pour induire des oscillations gamma dans le cerveau d'un spectateur (5) ; et
une deuxième logique de programme pour provoquer un affichage, après l'induction des oscillations gamma, pendant une deuxième période de temps, d'une deuxième image sur le dispositif d'affichage (15) après la première période de temps, la deuxième image comprenant un contenu à valence positive approprié au traitement du trouble lié à l'humeur.
